# EUROPÄISCHE PATENTSCHRIFT

(11) **EP 4 366 669 B1**
(45) Veröffentlichungstag und Bekanntmachung des Hinweises auf die Patenterteilung: **16.09.2026**
(21) Anmeldenummer: 22744150.8
(22) Anmeldetag: 01.07.2022
(51) Int. Cl.: A61F 5/01, A61F 13/06, A61F 13/10

(54) **BANDAGE FÜR DAS HANDGELENK ODER DAS SPRUNGGELENK**
BANDAGE FOR THE WRIST OR ANKLE JOINT
BANDAGE POUR LE POIGNET OU LA CHEVILLE

(30) Priorität: 06.07.2021 DE 102021207095
(43) Veröffentlichungstag der Anmeldung: 15.05.2024
(73) Patentinhaber: Bauerfeind AG, 07937 Zeulenroda-Triebes (DE)
(72) Erfinder: BAUERFEIND, Hans B., 07937 Zeulenroda-Triebes (DE); BOGUSCH, Toni, 07937 Zeulenroda-Triebes (DE)
(74) Vertreter: Wohlfahrt, Jan Günther
(86) Internationale Anmeldenummer: PCT/EP2022/068301
(87) Internationale Veröffentlichungsnummer: WO 2023/280718

(56) Entgegenhaltungen:
- DE-U1- 202014 005 449
- US-A- 4 495 942
- US-A1- 2010 069 802
- US-A1- 2020 093 628

## Beschreibung

Die vorliegende Erfindung betrifft eine Gelenkbandage für ein Handgelenk oder ein Sprunggelenk mit einem Gurtelement.

Extremitätenendgelenke, also Handgelenk und Sprunggelenk, die das Zygopodium, also den Unterarm bzw. den Unterschenkel, mit dem Basipodium und Metapodium, also der Hand oder dem Fuß, beweglich Verbinden, sind beispielsweise beim Sport oder durch Alterserscheinungen anfällig für eine Überlastung und degenerative Erkrankungen, was zu Schmerzen führen kann. Daher werden prophylaktisch oder therapeutisch diese Gelenke häufig bandagiert.

Im Stand der Technik sind die verschiedensten Handgelenksbandagen und Sprunggelenkbandagen bekannt. Diese basieren häufig auf einem schlauchförmigen gestreckt, wobei dem Gestrick ein das Handgelenk umlaufender kurzer Kompressionsgurt zugeordnet sein kann, wie in der DE 20 2012 004 652 U1 oder der EP 2090 273 A2 gezeigt. Die US 2020/093628 A1, die den Oberbegriff des Anspruchs 1 zeigt, offenbart eine schlauchförmige Fußgelenksbandage mit einem zugeordneten Gurt mit unterschiedlich dehnbaren Abschnitten, der um das Fußgelenk gewickelt werden kann. Häufig haben diese Bandagen auch ein zugeordneten Stabilisierungsstab, um die Flexionsbewegung festzulegen. Bei solchen Bandagen besteht immer eine Diskrepanz zwischen einem hohen Maß an Stabilisierung und gleichzeitig hoher Bewegungsmöglichkeit. Auch werden die Stabilisierungsstäbe häufig als störend empfunden. Insbesondere bei temporären sportlichen Aktivitäten wie Volleyball oder Handball, aber auch bei täglichen Aktivitäten wie Fahrradfahren oder Autofahren sind solche Stäbe hinderlich.

Der vorliegenden Erfindung liegt das technische Problem zugrunde Gelenkbandagen, insbesondere Handgelenksbandagen und Sprunggelenksbandagen, bereitzustellen, die eine verbesserte Funktionalität aufweisen, und die es insbesondere ermöglichen bei einem hohen Maß der Stabilisierung des Gelenks gleichzeitig eine hohe Bewegungsfreiheit zu gewährleisten. Dabei soll die Bandage dennoch möglichst einfach aufgebaut sein und dadurch auch eine gute Handhabbarkeit, insbesondere beim Anlegen der Bandage, gewährleisten.

Die vorliegende Erfindung löst das ihr zugrunde liegende technische Problem durch den Gegenstand der unabhängigen Ansprüche.

Die vorliegende Erfindung betrifft eine Gelenkbandage für ein Handgelenk oder ein Sprunggelenk, umfassend ein Gurtelement mit einem ersten Ende und einem zweiten Ende, wobei das Gurtelement mindestens zwei elastische Abschnitte und mindestens einen nicht elastischen Abschnitt zwischen den zwei elastischen Abschnitten aufweist, wobei das zweite Ende des Gurtelements am Gurtelement oder an einem Schlauchelement befestigt oder reversibel befestigbar ist, nach Anspruch 1.

Die vorliegende Erfindung betrifft auch eine Gelenkbandage für ein Handgelenk oder ein Sprunggelenk nach Anspruch 1, umfassend ein elastisches Schlauchelement und ein Gurtelement, wobei das Schlauchelement eine erste Schlauchhälfte mit einem ersten Ende und eine zweite Schlauchhälfte mit einem zweiten Ende aufweist, wobei das Gurtelement ein erstes Ende und ein zweites Ende aufweist, wobei das erste Ende des Gurtelements an der ersten Schlauchhälfte befestigt ist oder befestigbar ist, wobei das zweite Ende des Gurtelements am Gurtelement oder an der ersten Schlauchhälfte reversibel befestigbar ist, dadurch gekennzeichnet, dass im angelegten Zustand das erste Ende des Gurtelements an der ersten Schlauchhälfte befestigt ist und am Zygopodium positioniert ist und das Gurtelement vom ersten Ende aus distal über das Extremitäten-Endgelenk zum Basipodium verläuft, Basipodium oder Metapodium auf der Innenseite umläuft, dorsal über das Extremitäten-Endgelenk zum Zygopodium verläuft und sich dabei kreuzt, das Zygopodium umläuft und mit dem zweiten Ende im Bereich des Zygopodiums am Gurtelement oder an der ersten Schlauchhälfte reversibel befestigt ist.

Die vorliegende Erfindung betrifft auch eine Gelenkbandage für Extremitäten-Endgelenke, also für Handgelenke oder Sprunggelenke nach Anspruch 1, umfassend ein elastisches Schlauchelement und ein Gurtelement, wobei das Schlauchelement eine erste Schlauchhälfte mit einem ersten Ende und eine zweite Schlauchhälfte mit einem zweiten Ende aufweist, wobei das Gurtelement ein erstes Ende und ein zweites Ende aufweist, wobei das erste Ende des Gurtelements an der ersten Schlauchhälfte befestigt ist oder befestigbar ist, wobei das zweite Ende des Gurtelements am Gurtelement oder an der ersten Schlauchhälfte befestigt oder reversibel befestigbar ist, dadurch gekennzeichnet, dass das Gurtelement mindestens 40 cm lang ist.

Es zeigte sich, dass die Gurtelemente der erfindungsgemäßen Bandage so am Handgelenk oder Fußgelenk angelegt werden können, dass durch das Gurtelement selbst eine vorteilhafte Stabilisierung des Gelenks erreicht wird, sodass bei Bedarf auf einen Stabilisierungsstab verzichtet werden kann und die Gurtelemente trotz der Stabilisierung eine gute Bewegungsfreiheit erlauben. Die Bewegung der Hand oder des Fußes bis in die eingeschränkten/abgestützten Bereiche, die durch das Gurtelemente zugelassen werden, sind verbunden mit einer immer höher werdenden Arbeit um die durch die Bandage erzeugte Gegenkraft zu überwinden. Die Gurtelemente der erfindungsgemäßen Bandage erlauben das Anlegen an den Unterarm/Hand bzw. an den Unterschenkel/Fuß in einem vorteilhaften Verlauf, der zu einer deutlich erhöhten Stabilisierung im Vergleich zu einem einfach um das Gelenk verlaufenden Gurt auch ohne Stabilisierungsstab führt.

Im Zusammenhang mit der vorliegenden Erfindung wird unter einem Extremitätenendgelenk das Handgelenk und das Sprunggelenk, auch Fußgelenk genannt, zusammengefasst. Unter dem Zygopodium werden der Unterarm und der Unterschenkel zusammengefasst, unter Basipodium Handwurzel und Fußwurzel und unter Metapodium Mittelhand und Mittelfuß. Die vorliegende Erfindung betrifft Bandagen für Menschen aber auch für andere Säugetiere.

Die vorliegende Erfindung betrifft sowohl medizinische Gelenksbandagen als auch Sportbandagen. Die vorliegende Erfindung betrifft Handgelenksbandagen und/oder Sprunggelenksbandagen.

Die erfindungsgemäße Gelenkbandage kann mit einem Schlauchelement oder ohne einem Schlauchelement ausgestattet sein.

In einer ersten Ausführungsform ist die erfindungsgemäße Gelenkbandage ohne Schlauchelement vorgesehen und umfasst bevorzugt nur das Gurtelement.

In dieser ersten Ausführungsform betrifft die Erfindung insbesondere eine Gelenkbandage für ein Handgelenk oder ein Sprunggelenk, umfassend ein Gurtelement mit einem ersten Ende und einem zweiten Ende, wobei das Gurtelement mindestens zwei elastische Abschnitte und mindestens einen nicht elastischen Abschnitt zwischen den zwei elastischen Abschnitten aufweist, wobei das zweite Ende des Gurtelements am Gurtelement befestigt oder bevorzugt reversibel befestigbar ist.

Bevorzugt weist dabei das Gurtelement einen Fixierungsabschnitt auf, der um das Zygopodium, insbesondere den Unterarm oder den Unterschenkel, verlaufen kann und der Fixierungsabschnitt zwei miteinander verbindbare Bereiche aufweist, so dass der Fixierungsabschnitt durch das Verbinden der zwei Bereiche am Zygopodium, insbesondere am Unterarm oder am Unterschenkel fixierbar ist. Das Gurtelement kann sich so mittels Ringbildung /Ringbandage am Handgelenk auf sich selbst festlegen und bildet somit seinen eigenen "Anker" zur Fixierung des Gurtelements an sich selbst.

Bevorzugt weist dabei das Gurtelement ein erstes Ende und ein zweites Ende auf, wobei das erste Ende des Gurtelements durch den Fixierungsabschnitt gebildet wird und am Zygopodium befestigbar ist, wobei das zweite Ende des Gurtelements am Gurtelement reversibel befestigbar ist, wobei im angelegten Zustand das erste Ende des Gurtelements durch den Fixierungsabschnitt am Zygopodium positioniert ist und das Gurtelement vom ersten Ende aus distal über das Extremitäten-Endgelenk zum Basipodium verläuft, Basipodium oder Metapodium auf der Innenseite umläuft, dorsal über das Handgelenk oder Sprunggelenk zum Zygopodium verläuft und sich dabei kreuzt, das Zygopodium umläuft und mit dem zweiten Ende im Bereich des Zygopodiums am Gurtelement reversibel befestigt ist.

In einer alternativen Ausführungsform ist dem Gurtelement ein Schlauchelement zugeordnet. Solche Schlauchelemente sind dem Fachmann bekannt. Bevorzugt überbrückt das Schlauchelement das Handgelenk oder das Sprunggelenk.

Die Erfindung betrifft also auch eine Gelenkbandage für ein Handgelenk oder ein Sprunggelenk, umfassend ein Schlauchelement und ein Gurtelement mit einem ersten Ende und einem zweiten Ende, wobei das Gurtelement mindestens zwei elastische Abschnitte und mindestens einen nicht elastischen Abschnitt zwischen den zwei elastischen Abschnitten aufweist wobei das zweite Ende des Gurtelements am Gurtelement oder an einem Schlauchelement reversibel befestigbar ist.

Die Erfindung betrifft also auch eine Gelenkbandage, umfassend ein elastisches Schlauchelement und ein Gurtelement, wobei das Schlauchelement eine erste Schlauchhälfte mit einem ersten Ende und eine zweite Schlauchhälfte mit einem zweiten Ende aufweist, wobei das Gurtelement ein erstes Ende und ein zweites Ende aufweist, wobei das erste Ende des Gurtelements an der ersten Schlauchhälfte befestigt ist oder befestigbar ist, wobei das zweite Ende des Gurtelements am Gurtelement oder an der ersten Schlauchhälfte reversibel befestigbar ist, und wobei das Gurtelement mindestens 40 cm lang ist.

Die Erfindung betrifft auch eine Gelenkbandage für ein Handgelenk oder ein Sprunggelenk, umfassend ein elastisches Schlauchelement und ein Gurtelement, wobei das Schlauchelement eine erste Schlauchhälfte mit einem ersten Ende und eine zweite Schlauchhälfte mit einem zweiten Ende aufweist, wobei das Gurtelement ein erstes Ende und ein zweites Ende aufweist, wobei das erste Ende des Gurtelements an der ersten Schlauchhälfte befestigt ist oder befestigbar ist, wobei das zweite Ende des Gurtelements am Gurtelement oder an der ersten Schlauchhälfte reversibel befestigbar ist, dadurch gekennzeichnet, dass im angelegten Zustand das erste Ende des Gurtelements an der ersten Schlauchhälfte befestigt ist und am Zygopodium positioniert ist und das Gurtelement vom ersten Ende aus distal über das Extremitäten-Endgelenk zum Basipodium verläuft, Basipodium oder Metapodium auf der Innenseite umläuft, dorsal über das Extremitäten-Endgelenk zum Zygopodium verläuft und sich dabei kreuzt, das Zygopodium umläuft und mit dem zweiten Ende im Bereich des Zygopodiums am Gurtelement oder an der ersten Schlauchhälfte reversibel befestigt ist.

Bevorzugt ist das Schlauchelement aus Neopren, einem Gewebe oder einer Maschenware gebildet, beispielsweise aus einem Gestrick oder einem Gewirk. Bevorzugt weist das Schlauchelement eine proximale Öffnung auf. Distal kann das Schlauchelement entweder eine distale Öffnung aufweisen oder als Handschuh oder Strumpf ausgestaltet sein. Bevorzugt weist das Schlauchelement zusätzlich eine separate Öffnung für den Daumen oder den großen Zehen auf.

Bevorzugt weist die Gelenkbandage keinen Stabilisierungsstab auf. Es zeigte sich überraschenderweise, dass bei der erfindungsgemäßen Gelenksbandage, insbesondere mit Schlauchelement, auf einen Stabilisierungsstab verzichtet werden kann, und dennoch durch die durch das erfindungsgemäße Gurtelement mögliche Gurtführung eine ausreichende Stabilität erreicht wird. Es kann jedoch vorgesehen sein, bei Bedarf zusätzlich die Möglichkeit vorzusehen einen Stabilisierungsstab in die Gelenksbandage, insbesondere das Schlauchelement, zumindest zeitweise einzusetzen.

Die Gelenkbandage kann am Schlauchelement ein Spannelement aufweisen. Dieses Spannelement kann dazu genutzt werden um den Körper des Schlauchelements vor zu formen und/oder auf zu spannen, sodass das Schlauchelement leichter angezogen werden kann. Auch wenn das Spannelement als Stab ausgebildet sein kann handelt es sich dabei nicht um einen Stabilisierungsstab, da das Spannelement durch seine Form und Positionierung an der Gelenksbandage keine Stabilisierungsfunktion des Gelenks übernimmt, übernehmen soll und übernehmen kann, sondern allein verhindert, dass sich das Schlauchelement nicht in unerwünschter Weise verformt, insbesondere nicht gestaucht werden kann.

Das Schlauchelement kann bei Bedarf mit Pelotten ausgerüstet sein.

Bevorzugt ist das Gurtelement mindestens 40 cm lang. Ein Gurtelement, das mindestens 40 cm lang ist, erlaubt im angezogenen Zustand der Gelenksbandage einen Gurtverlauf um Unterarm/Unterschenkel, Hand/Fuß und dazwischen liegendem Gelenk gemäß der vorliegenden Erfindung.

Das erste Ende des Gurtelements ist entweder am Schlauchelement oder an sich selbst im Bereich des Zygopodiums befestigt. Die Befestigung des ersten Gurtelements kann reversibel sein, beispielsweise über einen Klettverschluss oder über Druckknöpfe, oder das Gurtelement kann mit seinem ersten Ende dauerhaft am Schlauchelement befestigt sein oder an sich selbst dauerhaft befestigt sein, sodass es eine Schlaufe bildet. Das Gurtelement verläuft dann um das Extremitätenendgelenk wie vorliegend beschrieben.

Erfindungsgemäß ist das zweite Ende des Gurtelements am Gurtelement oder an einem Schlauchelement reversibel befestigbar. Die reversible Befestigung ermöglicht ein Lösen der Befestigung und in vorteilhafter Weise ein Spannen des Gurtelements, beispielsweise wenn am Gurtelement und/oder am Schlauchelement Bereiche vorgesehen sind, die eine unterschiedliche Positionierung des zweiten Endes des Gurtelements zulassen, beispielsweise Klettverschlussbereiche.

Erfindungsgemäß weist das Gurtelement mindestens zwei elastische Abschnitte und mindestens einen nicht elastischen Abschnitt zwischen den zwei elastischen Abschnitten auf. Bevorzugt weist das Gurtelement sich abwechselnde elastische und nicht elastische Abschnitte auf. Bei einem vollkommen elastisch dehnbaren Gurtelement kann es zu unangenehmen Einschnürungen vor allem im Bereich des Daumens bzw. des großen Zehs kommen. Bei einem vollkommen unelastischen Gutelement wird die Hand oder der Fuß zu stark fixiert und eine Einstellbarkeit der Stabilisierung ist nicht gewährleistet. Eine Kombination von elastischen und nicht elastischen Abschnitten im Gurtelement ermöglicht in vorteilhafter Weise die Überwindung dieser Probleme.

Bevorzugt sind die elastischen Abschnitte des Gurtelements elastisch dehnbar. Die elastisch dehnbaren Abschnitte ermöglichen es, dass das Gurtelement sich an bestimmten Abschnitten ausdehnen kann, wenn es beim Anliegen der Bandage durch Ziehen des Gurtelements gespannt wird und das Gurtelement beim Ausziehen der Bandage wieder in einen entspannten Grundzustand zurückkehrt.

Bevorzugt verläuft mindestens ein elastischer Abschnitt des Gurtelements nicht orthogonal zur der Schwenkachse des Handgelenks oder des Sprunggelenks, die beeinflusst werden soll. Bevorzugt verläuft mindestens ein elastischer Abschnitt des Gurtelements in einem Winkel von 30 bis 180 Grad, mehr bevorzugt von 35 bis 170 Grad, besonders bevorzugt von 40 Grad bis 50 Grad, insbesondere von etwa 45 Grad zur der Schwenkachse.

Bevorzugt verläuft ein elastischer Abschnitt des Gurtelements bis zum zweiten Ende des Gurtelements oder bis zu einem Befestigungsbereich am zweiten Ende des Gurtelements.

Bevorzugt verläuft mindestens ein elastischer Abschnitt des Gurtelements über dem Handrücken oder über der Handfläche beziehungsweise über dem Fußrücken oder der Fußsohle.

Bevorzugt verläuft mindestens ein elastischer Abschnitt des Gurtelements über dem Handrücken oder der Handfläche beziehungsweise über dem Fußrücken oder der Fußsohle und/oder mindestens ein nicht elastischer Abschnitt verläuft an den Handkanten oder Fußkante. Bevorzugt verläuft mindestens ein nicht elastischer Abschnitt an den Handkanten oder Fußkante.

Dem Fachmann sind geeignete Materialen für das Gurtelement und für die elastischen und unelastischen Abschnitte des Gurtelements bekannt.

Erfindungsgemäß weist das Gurtelement mindestens einen verjüngten Abschnitt auf. Der verjüngte Abschnitt ist auf dem Gurtelement so positioniert, dass er im angezogenen Zustand im Bereich des Daumens bzw. des großen Zehs liegt. Die Verjüngung kommt also dabei in dem Bereich zwischen Daumen und Zeigefinger bzw. zwischen Großzehe und daneben liegendem Zeh zum Liegen und passt sich durch die Verjüngung in vorteilhafter Weise dem schmalen Fingerzwischenraum bzw. Zehenzwischenraum an. Die Verjüngung kann in Form eines eingeschnittenen Bogens ausgestaltet sein oder durch einen schmalen Abschnitt des Gurtbands gebildet werden. Alternativ kann das Gurtelement an dieser Position auch ein Loch aufweisen, durch das der Daumen oder der große Zeh gesteckt werden kann.

Erfindungsgemäß ist der verjüngte Abschnitt unelastisch.

Erfindungsgemäß weist das Gurtelement mindestens einen Abschnitt in Form eines Parallelogramms oder einer Krümmung oder einen Knick auf. Bevorzugt weist das Gurtelement mindestens einen Abschnitt in Form eines Parallelogramms auf. Der Abschnitt in Form eines Parallelogramms oder einer Krümmung bzw. Form eines Knicks bildet im Verlauf des Gurtelements eine Abschrägung, die zu einem besonders angepassten Verlauf des Gurtelements im Gelenkbereich führt.

Erfindungsgemäß ist der Abschnitt in Form eines Parallelogramms oder einer Krümmung oder eines Knicks unelastisch.

Erfindungsgemäß befindet sich zwischen dem unelastischen verjüngten Abschnitt und dem unelastischen Abschnitt in Form eines Parallelogramms oder einer Krümmung oder eines Knicks ein elastischer Abschnitt.

Erfindungsgemäß weist das Gurtelement mindestens einen verjüngten Abschnitt und mindestens einen Abschnitt in Form eines Parallelogramms oder einer Krümmung oder einen Knick auf.

Erfindungsgemäß weist das Gurtelement aufeinanderfolgend einen ersten elastischen Abschnitt, einen ersten nicht elastischen Abschnitt, einen zweiten elastischen Abschnitt, einen zweiten nicht elastischen Abschnitt und einen dritten elastischen Abschnitt auf, wobei der erste nicht elastische Abschnitt einen Abschnitt in Form eines Parallelogramms oder einer Krümmung oder einen Knick aufweist und wobei der zweite nicht elastische Abschnitt eine Verjüngung aufweist.

Bevorzugt ist dem ersten elastischen Abschnitt noch ein nicht elastischer Vorabschnitt zugeordnet.

Bevorzugt umfasst das Gurtelement mindestens zwei reversibel miteinander verbindbare Gurtteileilelemente.

Bevorzugt umfasst das Gurtelement ein erstes Gurtteileilelement mit dem ersten Ende und ein zweites Gurtteilelement mit dem zweiten Ende, wobei das zweite Gurtteilelement elastisch ist.

Bevorzugt sind das erste Gurtteilelement und das zweite Gurtteilelement reversibel über ein drittes Ende am ersten Gurtteilelement und ein viertes Ende am zweiten Gurtteilelement miteinander verbindbar, wobei bevorzugt das vierte Ende am zweiten Gurtteilelement ein Verbindungselement umfasst, mit dem das zweite Gurtteilelement ohne das erste Gurtteilelement am Schlauchelement reversibel befestigt werden kann. In dieser bevorzugten Ausführungsform kann das zweite Gurtteilelement ohne das erste Gurtteilelement verwendet werden, beispielsweise als um das Handgelenk oder das Fußgelenk gelegte Manschette. Damit kann die Bandage beispielsweise in vorteilhafter Weise dem Reha-Verlauf des Patienten oder der Bewegungsintensität des Trägers unabhängig von einer Reha angepasst werden, indem in einem ersten Therapieschritt beide Gurtteilelemente wie vorliegend beschrieben verwendet werden, dann in einem zweiten fakultativen Therapieschritt nur das zweite Gurtteilelement als Manschette um das Handgelenk oder das Fußgelenk verwendet wird und in einem dritten fakultativen Therapieschritt das Schlauchelement als Kompression Manschette verwendet wird. Eine solche Auf- und Abrüstung der Bandage mit dem ersten Gurtelement ist aber auch anderweitig vorteilhaft, zum Beispiel beim Sport oder beim Autofahren.

Natürlich kann das Gurtelement auch einstückig ausgebildet sein, beispielsweise wenn nur eine Prophylaxe oder eine Therapie mit dem hier beschriebenen Gurtverlauf gewünscht ist.

Eine weitere Ausführungsform der erfindungsgemäßen Gelenkbandage für ein Handgelenk oder ein Sprunggelenk umfasst ein Gurtelement mit einem ersten Ende und einem zweiten Ende, wobei das Gurtelement mindestens zwei elastische Abschnitte und mindestens einen nicht elastischen Abschnitt zwischen den zwei elastischen Abschnitten aufweist wobei das zweite Ende des Gurtelements am Gurtelement befestigt oder reversibel befestigbar ist. Bei dieser Ausführungsform kann ein Schlauchelement vorgesehen sein oder nicht. Bei dieser Ausführungsform bildet das Gurtelement eine Schlaufe, die um die Hand oder den Fuß herum verlaufen kann und dabei zwischen dem Daumen/großen Zeh und dem Zeigefinger/zweiter Zeh verläuft. Der elastische Gurtabschnitt ist dabei an der Handinnenfläche oder der Handaußenfläche beziehungsweise Fußinnenfläche oder der Fußaußenfläche positioniert, die zwei unelastischen Abschnitte an den Handkanten beziehungsweise Fußkanten, so dass eine Positionssicherung gegeben ist und ein Einschneiden verhindert wird. Die Schlaufe wird durch eine Verbindung des zweiten Endes des Gurtelements an einem Mittelabschnitt des Gurtelements gebildet. Von diesem Verbindungsbereich zum ersten Ende des Gurtelements hin verläuft das Gurtelement über das Gelenk zum Unterarm oder Unterschenkel. Am Unterarm oder Unterschenkel ist das erste Ende des Gurtelements entweder mit einem Schlauchelement verbunden, das auch relativ kurz sein kann, oder das erste Ende des Gurtelements bildet einen wie oben beschriebenen Fixierungsabschnitt, mit dem das Gurtelement direkt am Unterarm oder Unterschenkel befestigt werden kann. Der Verbindungsbereich der Schlaufe kann entweder auf der Oberseite oder der Unterseite der Hand oder des Fußes positioniert sein, je nachdem welche Bewegungsrichtung limitiert werden soll.

Der Fachmann kann ohne weiteres die vorliegen beschriebenen Elemente der verschiedenen Ausführungsformen der erfindungsgemäßen Gelenkbandage kombinieren.

Weiter bevorzugte Ausführungsformen ergeben sich aus den Beispielen, den Figuren und den Unteransprüchen, ohne dass diese einschränkend zu verstehen wären.

Die Bewegung der Hand oder des Fußes bis in die eingeschränkten/abgestützten Bereiche, die durch das Gurtelemente zugelassen werden, sind verbunden mit einer immer höher werdenden Arbeit um die durch die Bandage erzeugte Gegenkraft zu überwinden. Die aufzubringende Kraft wird immer größer bis zu dem Punkt, an dem keine weitere Bewegung in die eingeschränkte/abgestützte Richtung mehr möglich ist. Ab einer bestimmten Gelenkstellung wird durch die Gurtführung eine Gegenkraft erzeugt, ähnlich wie bei einem Lasso, welches sich unter Belastung weiter zuzieht und somit einen Gegenstand stärker fixiert. Somit kann in der gezeigten Ausführungsform in vorteilhafter Weise auf einen Stabilisierungsstab verzichtet werden.

Es zeigen
- Figur 1: eine erfindungsgemäße ausführungsform einer Handgelenksbandage mit Schlauchelement
- Figur 2: den ersten Teilabschnitt eines erfindungsgemäßes Gurtelements
- Figur 3: den zweiten Teilabschnitt des Gurtelements von Figur 2
- Figur 4: zwei alternative Ausführungsformen des Gurtelements
- Figur 5: zeigt den zweiten Teilabschnitt des Gurtelements aus Figur 3
- Figuren 6 - 10: zeigen verschiedene Schritte beim Befestigen des Gurtelements an das Schlauchelement
- Figur 11: zeigt die Krafteinleitung bei Bewegung der Hand
- Figuren 12 - 16: zeigen schematisch verschiedene Ansichten einer erfindungsgemäßen Sprunggelenkbandage
- Figur 17: zeigt eine alternative Ausführungsform der Handgelenksbandage
- Figur 18: zeigt eine weitere Verwendungsmöglichkeit der alternativen Ausführungsform aus Figur 17

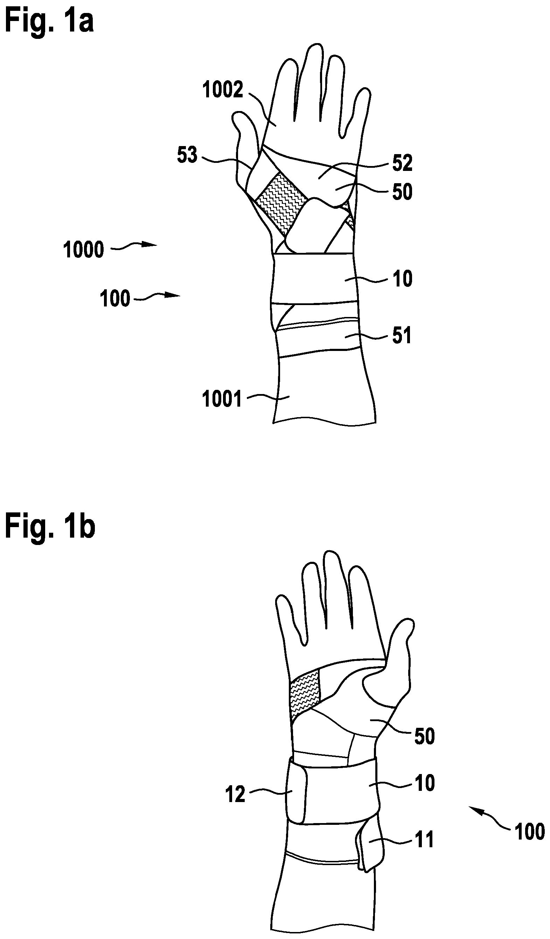

Figur 1 zeigt eine erfindungsgemäße Handgelenksbandage (100) in der Ausführungsform mit Schlauchelement (50) an einem rechten Handgelenk (1000). An dem Schlauchelement (50) ist ein erfindungsgemäßes Gurtelement (10) befestigt, das zur Stabilisierung des Handgelenks (1000) um den an das Handgelenk (1000) angrenzenden Bereich des Unterarms (1001), das Handgelenk (1000) und die Hand (1002) gelegt ist. Figur 1a zeigt die Handgelenksbandage (100) am Handrücken und Figur 1b zeigt die Handgelenksbandage (100) an der Handinnenfläche. Das Schlauchelement (50) eine erste Schlauchhälfte mit einem ersten Ende (51) und eine zweite Schlauchhälfte mit einem zweiten Ende (52) mit zusätzlicher Öffnung (53) für den Daumen, wobei das Gurtelement (10) ein erstes Ende (11) und ein zweites Ende (12) aufweist, wobei das erste Ende (11) des Gurtelements (10) an der ersten Schlauchhälfte (51) über einen Klettverbindung befestigt ist, wobei das zweite Ende (12) des Gurtelements (10) am Gurtelement (10) oder an der ersten Schlauchhälfte (51) über eine Klettverbindung reversibel befestigbar ist und vorliegend am Gurtelement (10) befestigt ist. Das erste Ende des Gurtelements (10) ist an der ersten Schlauchhälfte (51) befestigt ist und am Unterarm (1001) positioniert. Das Gurtelement (10) verläuft vom ersten Ende aus distal das Handgelenk (1000) querend zum Handrücken der Hand (1002), umläuft die Hand (1002) auf der Handinnenseite, verläuft dann im Zwischenraum zwischen Daumen und Zeigefinger. Von dort verläuft das Gurtelement (10) dorsal wieder über das Handgelenk (1000) zum Unterarm (1001) und kreuzt sich dabei. Das Gurtelement (10) umschlingt dann den Unterarm (1001) und ist mit dem zweiten Ende (12) im Bereich des Unterarms nahe dem Handgelenk an sich selbst festgeklettet. Dabei kann durch spannen des Gurtelements (10) und entsprechender Positionierung des zweiten Endes (12) die durch das Gurtelement (10) wirkende Kraft eingestellt werden. Es ist bei dieser Bandage kein Stabilisierungsstab nötig.

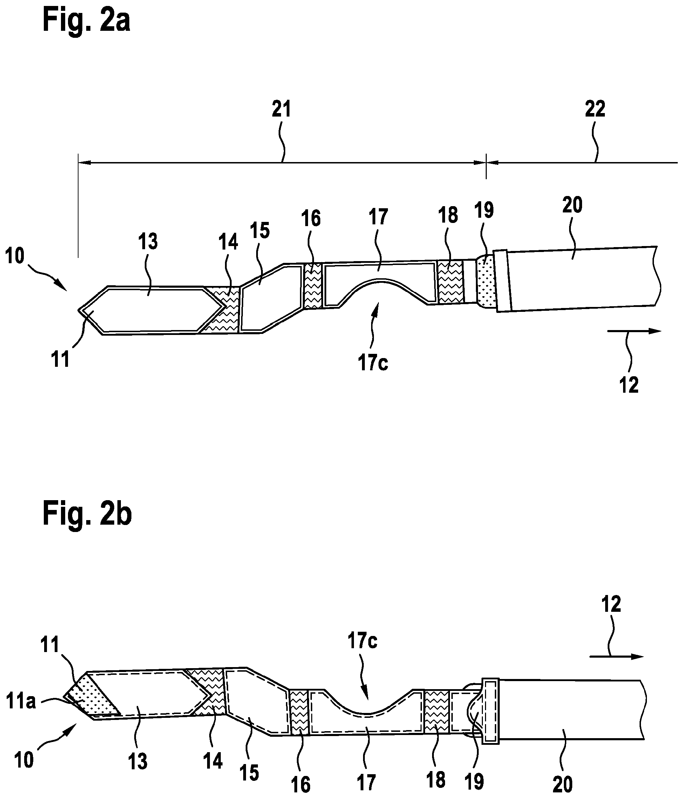

Figur 2 zeigt das erfindungsgemäße Gurtelement (10) der Gelenksbandage aus Figur 1. Figur 2A zeigt die Außenseite des Gurtelements (10) und Figur 2B die Innenseite des Gurtelements (10). Das Gurtelement (10) ist im ungedehnten Zustand etwa 64 cm lang und hat mehrere abwechselnde elastische und unelastische Abschnitte. Am ersten Ende (11) befindet sich ein unelastischer Vorabschnitt (13), mit dem das Gurtelement am Schlauchelement über ein Klettelement (11a) befestigt werden kann. Es folgt ein erster elastischer Abschnitt (14). Es folgt ein erster unelastischer Abschnitt (15), der in Form eines Parallelogramms ausgestaltet ist und somit zu einer Verschiebung des Gurtverlaufs führt. Es schließt sich ein kurzer zweiter elastischer Abschnitt (16) an, gefolgt von einem zweiten unelastischen Abschnitt (17), der eine Verjüngung in Form eines ausgeschnittenen Bogens (17c) aufweist. Im angelegten Zustand liegt diese Verjüngung zwischen Daumen und Zeigefinger, sodass das Gurtelement (10) in diesem Bereich bequem anliegt. Es folgt wieder ein kurzer dritter elastischer Abschnitt (18), der über eine Klettverbindung (19) mit einem langen vierten elastischen Abschnitt (20) verbunden ist. Der vierte elastische Abschnitt (20) endet am nicht mehr sichtbaren zweiten Ende (12) des Gurtelements (10) mit einem Klettelement.

Durch die Klettverbindung (19) lässt sich das Gurtelement (10) in ein erstes Teilelement (21) und ein zweites Teilelement (22) teilen.

Die elastischen Abschnitte (14,16,18,20) des Gurtelements (10) ermöglichen die notwendige Dehnung des Gurtelements (10) in angelegtem Zustand, die unelastischen Gurtabschnitte (13,15,17,19) sorgen für die nötige Krafteinleitung der Bandage und tragen somit zur Stabilisierung des Handgelenks bei. Die unelastischen Abschnitte dienen dabei insbesondere dazu, die Handbewegung in ungewünschte, insbesondere ungesunde Positionen zu erschweren oder zu verhindern.

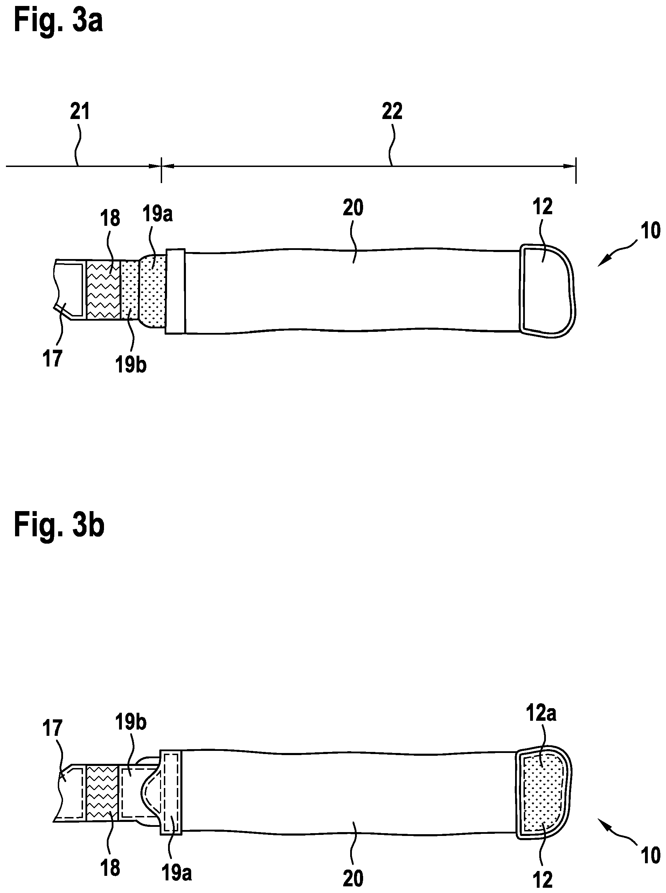

Figur 3 zeigt das erfindungsgemäße Gurtelement (10) der Gelenksbandage aus Figur 1. Figur 3A zeigt die Außenseite des Gurtelements (10) und Figur 3B die Innenseite des Gurtelements (10). Zu sehen ist der gesamte zweite Teilabschnitt (22) mit dem vierten elastischen Abschnitt (20), der am zweiten Ende (12) des Gurtelements (10) und dem dortigen Klettelement (12a) endet. Der vierte elastische Abschnitt (20) ist mit einem Klettelement (19a) mit dem Klettelement (19b) des ersten Teilabschnitts (21), der nicht ganz gezeigt ist, sondern bei dem nur der dritte elastische Abschnitt (18) und das Ende des zweiten unelastischen Abschnitts (17) zu sehen sind, reversibel verbunden.

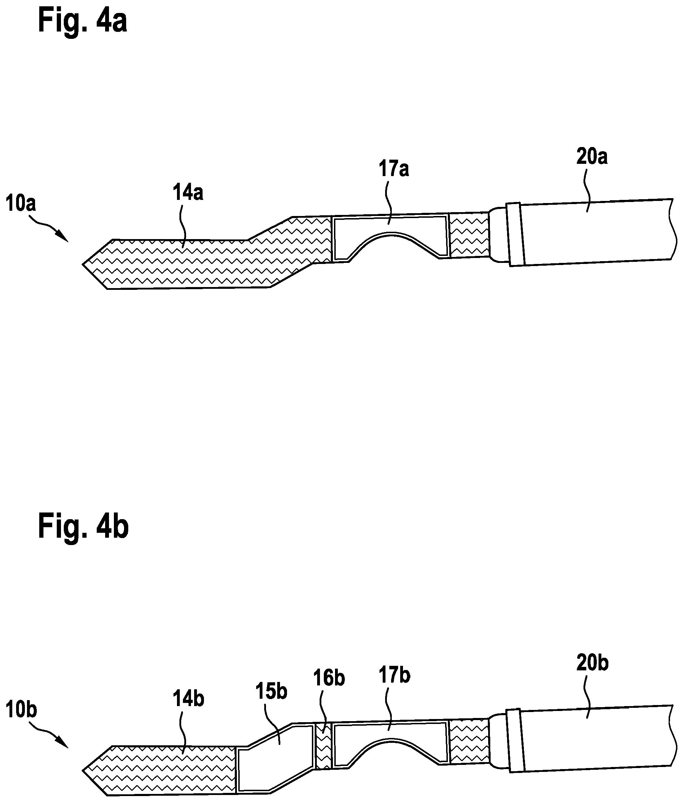

Figur 4 zeigt schematisch eine erste (10a) und eine zweite (10b) alternative und vereinfachte Ausführungsform des Gurtelements aus den Figuren 2 und 3. In Figur 4a umfasst das Gurtelement (10a) nur einen ersten elastischen Abschnitt (14a), einen zweiten elastischen Abschnitt (20a) und einen dazwischenliegenden unelastischen Abschnitt (17a) mit der Verjüngung für den Fingerzwischenraum. Figur 4b zeigt eine Ausführungsform des Gurtelements (10b) mit einem ersten elastischen Abschnitt (14b), einem als Parallelogramm ausgebildeten ersten unelastischen Abschnitt (15b) einem kurzen elastischen Zwischenabschnitt (16b), einem zweiten unelastischen Abschnitt (17b) mit Verjüngung und einem elastischen Endabschnitt (20b).

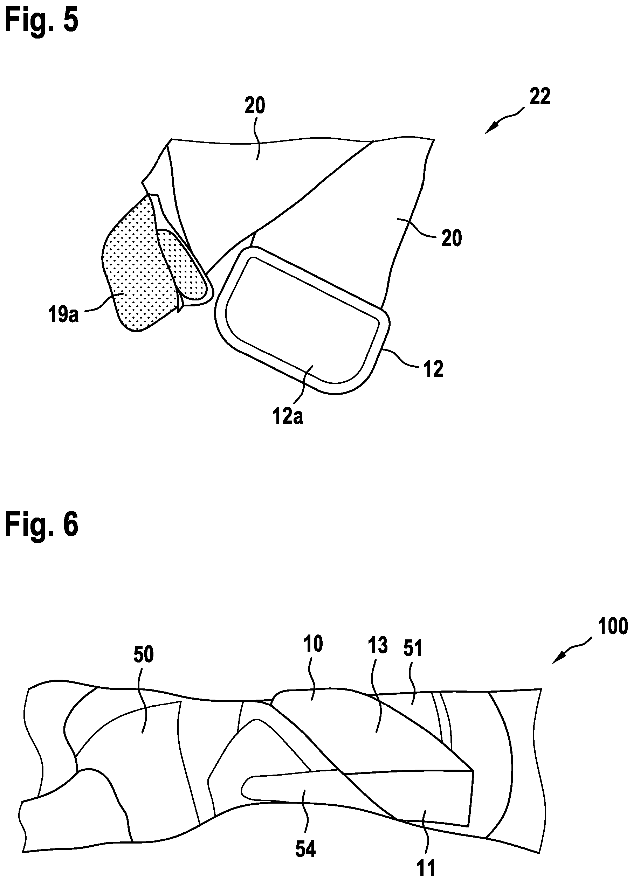

Figur 5 zeigt den zweiten Teilabschnitt (22) des Gurtelements mit dem vierten elastischen Abschnitt (20) an dessen einem Ende sich ein Klettelement (19a) mit Fischmaulverklettung zum sicheren Verbinden des zweiten Teilabschnitts (22) mit dem ersten Teilabschnitt befindet und sich am anderen Ende das zweite Ende (12) des Gurtelements mit einem Klettelement (12a) zur Befestigung am Gurtelement oder am Schlauchelement befindet.

Die Figuren 6-10 zeigen verschiedene Schritte beim Anlegen des Gurtelements (10) an dem Schlauchelement (50) der erfindungsgemäßen Gelenkbandage (10).

In Figur 6 ist ein erster Schritt gezeigt, bei dem das Gurtelement (10) an der ersten Hälfte (51) des Schlauchelements (50) über ein Klettelement des ersten Endes (11) des Gurtelements (10) befestigt wird. Der unelastische Vorabschnitt (13) verläuft schräg distal in Richtung Handgelenk. Zu sehen ist auch ein in das Gestrick eingebrachte oder auf das Gestrick aufgebrachte Spannelement (54), dass ein zusammenziehen des gestreckten Schlauchs verhindert, aber nicht zur Stabilisierung des Gelenks beiträgt.

Das erste Ende (11) des Gurtelements (10) wird so weit wie möglich vom Drehpunkt des Handgelenks entfernt befestigt. Dieser Festlegepunkt kann somit einen großen Hebel erzeugen, der die Flexionsbewegung sehr gut einschränkt. Das Einstellen der Vorspannung des Gurtsystems ist bereits an diesem Punkt durch eine mehr oder weniger starke dorsale Extension möglich. Je weiter der Anwender in diesem Schritt in Dorsalextension ist, desto mehr wird die Hand stabilisiert. Je stärker das Grundelement (10) gespannt wird, desto effektiver wird dem Abknicken/Herunterfallen der Hand entgegengewirkt.

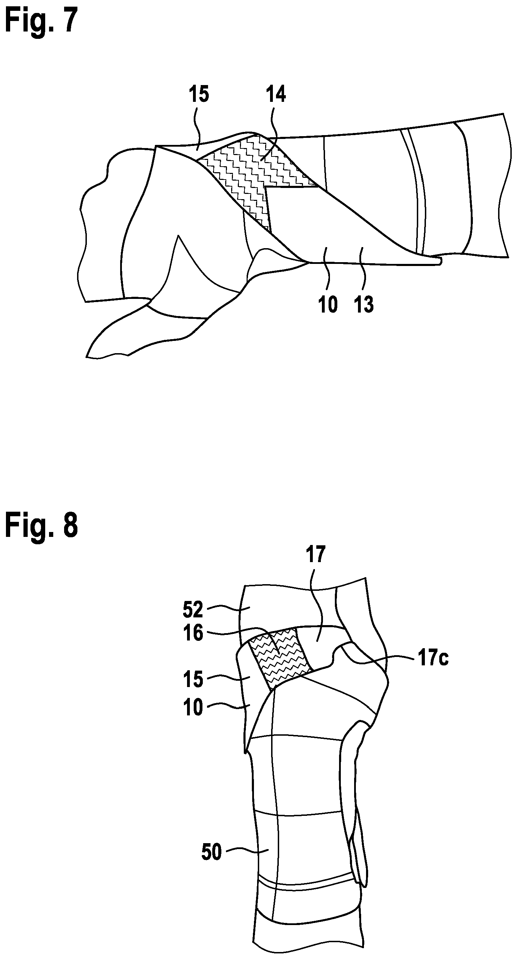

Figur 7 zeigt wie das Gurtelement (10) weiter mit dem unelastischen Vorabschnitt (13) und dem ersten elastischen Abschnitt (14) auf dem Handrücken sowie dem ersten unelastischen Abschnitt (15) an der Handaußenkante über den Handrücken geführt wird. Das Gurtelement (10) wird möglichst nahe an das Fingergrundgelenk des kleinen Fingers in die Handinnenfläche umgelenkt. Dies führt zu einer besseren Kraftübertragung zum Handgelenk. Somit entsteht eine maximale Hebelwirkung. Zeitgleich wird die Lage des Gurtelements (10) am Fingergrundgelenk als nicht störend empfunden, da der erste unelastische Abschnitt (15) als nicht einschneidend empfunden wird. Der erste elastische Abschnitt (14) am Handrücken beschränkt zudem in geringem Maße die Bewegung in radialer Richtung.

Figur 8 zeigt die Handinnenseite, bei der das Grundelement (10) von der linken Seite mit dem ersten unelastischen Abschnitt (15) kommt und die Handfläche und die zweite Hälfte (52) des Schlauchelements (50) mit dem zweiten elastischen Abschnitt (16) durchläuft. Es folgt der zweite unelastische Abschnitt (17), der mit seiner bogenförmigen Verjüngung (17c) zwischen Daumen und Zeigefinger hindurch in Richtung Handrücken gewickelt wird. Trotz der Verjüngung (17c) ist dieser Abschnitt breit genug und schneidet nicht ein, da er nicht elastisch ist. Auftretende Zuglasten bei Flexion und Spreizung werden auf eine größtmögliche Fläche verteilt. Der zweite unelastische Abschnitt (17) sorgt darüber hinaus für eine wünschenswerte Schonhaltung des Daumens.

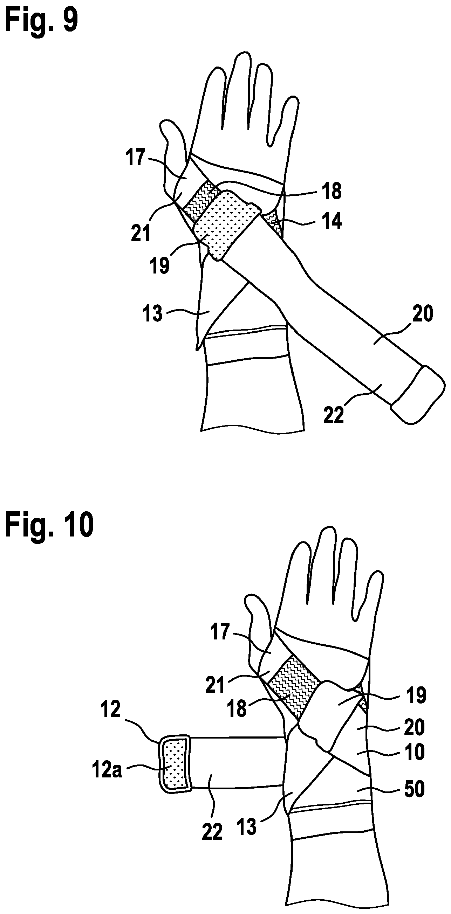

In Figur 9 ist die Handaußenseite zu sehen. Dem zweiten unelastischen Abschnitt (17) folgt der dritte elastische Abschnitt (18). Dieser lässt eine Bewegung in radial und eingeschränkt in ulnar zu und trägt weiterhin zur Lastaufnahme bei Flexion bei. Das erste Teilelement (21) bildet im Handbereich somit ein Dreieck, dass die Flexionsbewegung abfängt. Das erste Teilelement (21) endet somit am Handrücken und ist über eine Klettverbindung (19) mit dem zweiten Teilelement (22), das den vierten elastischen Abschnitt (20) umfasst, verbunden.

Es ist zu sehen, wie der dritte elastische Abschnitt (18), das Klettelement (19) und der vierte elastische Abschnitt (20) den unelastischen Vorabschnitt (13) und den ersten elastischen Abschnitt (14) kreuzen.

In Figur 10 ist zu sehen, wie der vierte elastische Abschnitt (20) um die ulnare Kannte des Unterarms herumgeschlagen wird. Durch dieses radiale Anlegen des zweiten Teilelements (22) kann eine zusätzliche Kompression und Stabilisierung hervorgerufen werden. Dies wirkt sich auch stabilisierend und entlastend auf den Karpaltunnel und das Karpalband aus. Das zweite Teilelement (22) kann am zweiten Ende (12) mit dem dortigen Klettelement (12a) am Gurtelement (10) oder am Schlauchelement (50) befestigt werden, sodass ein Gurtelementverlauf wie in Figur 1 gezeigt erhalten wird.

Durch die Aufteilung in elastische Abschnitte und unelastische Abschnitte wird die Krafteinleitung bei palmarer Flexionsbewegung eingeschränkt, wobei eine Einschränkung der Handbewegung in dorsaler Extension nicht gegeben ist. Die Hand lässt sich somit uneingeschränkt in diese Richtung bewegen.

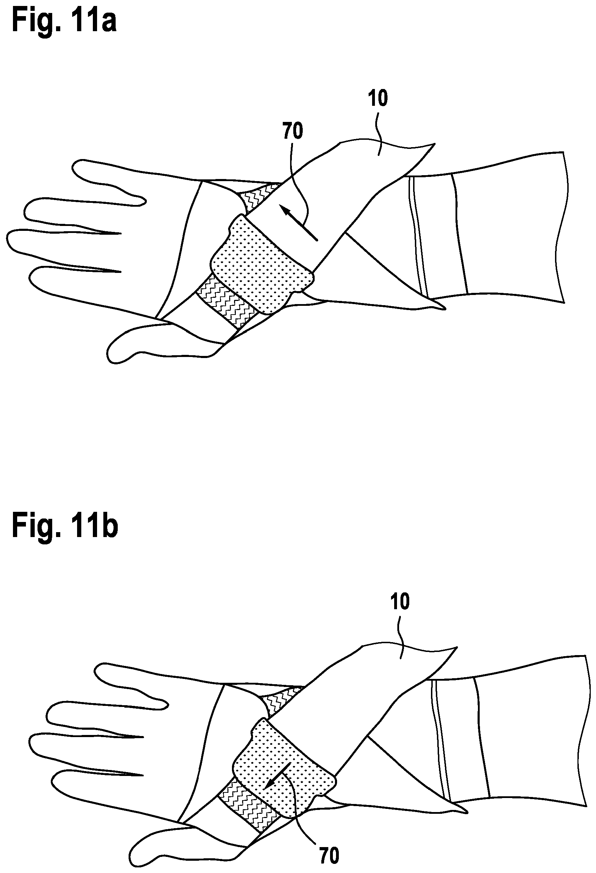

Figur 11 zeigt die Krafteinleitung (70) des Gurtelements (10) bei einer Handbewegung nach radial (Figur 11a) oder nach ulnar (Figur 11b). Bei einer Bewegung nach radial wird die Kraft durch den ersten elastischen Abschnitt abgefedert. Dieser lässt die Bewegung in radialer Richtung zu und schränkt diese lediglich stabilisierend ein. Bei einer Handbewegung in Richtung ulnar wird die Kraft durch den dritten elastischen Abschnitt und den vierten elastischen Abschnitt abgefedert. Diese lassen die Bewegung in ulnar Richtung zu und schränken diese lediglich stabilisierend ein.

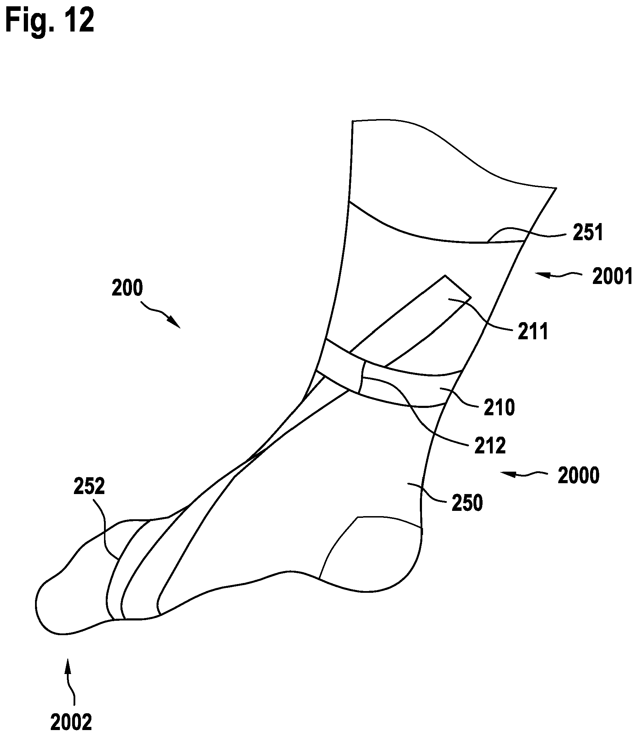

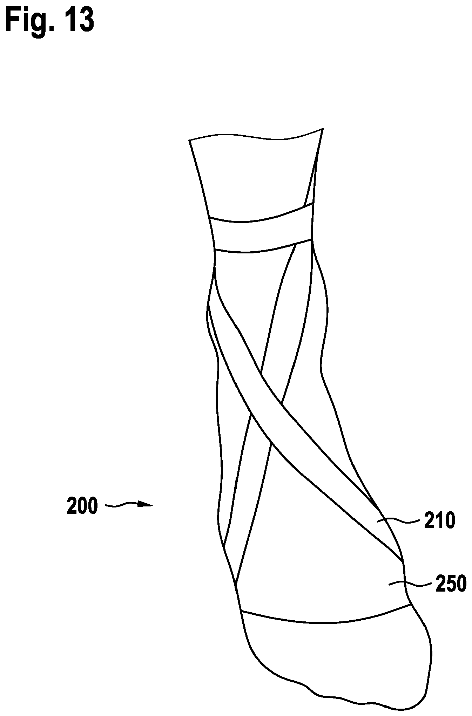

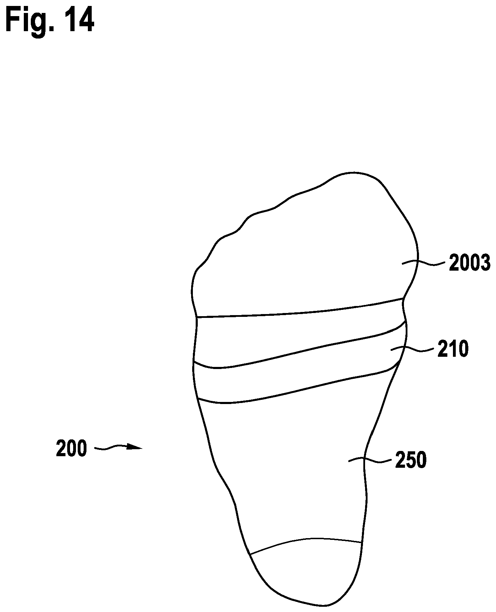

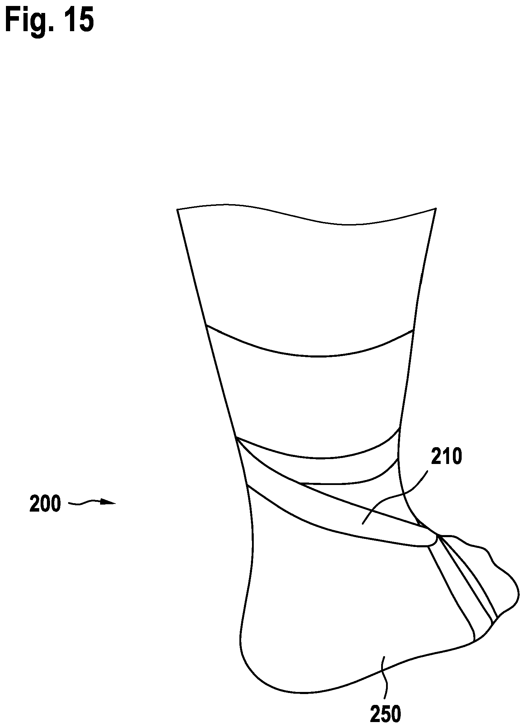

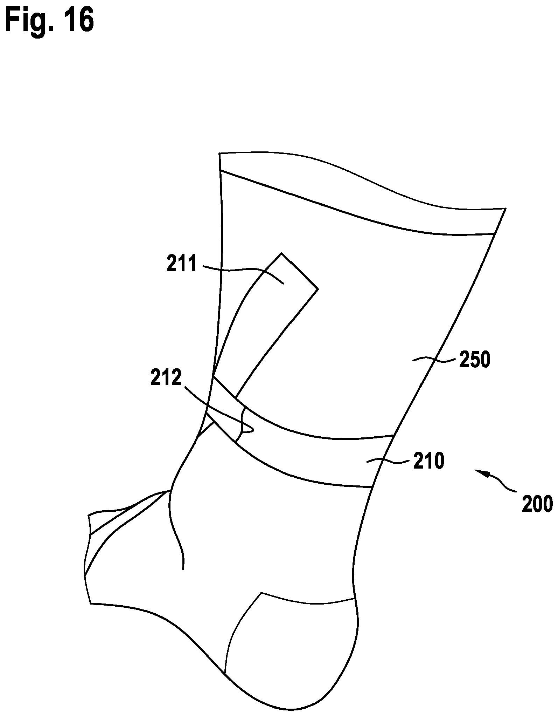

Die Figuren 12 bis 16 zeigen schematisch eine erfindungsgemäße Sprunggelenksbandage (200) in der Ausführungsform mit Schlauchelement (250) an einem rechten Sprunggelenk (2000). An dem Schlauchelement (250) ist ein erfindungsgemäßes Gurtelement (210) befestigt, das zur Stabilisierung des Sprunggelenks (2000) um den an das Sprunggelenk (2000) angrenzenden Bereich des Unterschenkels (2001), das Sprunggelenk (2000) und den Fuß (2002) gelegt ist. Das Schlauchelement (250) eine erste Schlauchhälfte mit einem ersten Ende (251) und eine zweite Schlauchhälfte mit einem zweiten Ende (252), wobei das Gurtelement (210) ein erstes Ende (211) und ein zweites Ende (212) aufweist, wobei das erste Ende (211) des Gurtelements (210) an der ersten Schlauchhälfte (251) über einen Klettverbindung befestigt ist, wobei das zweite Ende (212) des Gurtelements (210) am Gurtelement (210) oder an der ersten Schlauchhälfte (251) über eine Klettverbindung reversibel befestigbar ist und vorliegend am Gurtelement (210) befestigt ist. Das erste Ende des Gurtelements (210) ist an der ersten Schlauchhälfte (51) befestigt ist und am Unterschenkel (2001) positioniert. Das Gurtelement (210) verläuft vom ersten Ende aus distal das Sprunggelenk (2000) querend zum Fußrücken des Fußes (2002), umläuft den Fuß (2002) auf der Fußsohlenseite (2003). Von dort verläuft das Gurtelement (210) dorsal wieder über das Sprunggelenk (2000) zum Unterschenkel (2001) und kreuzt sich dabei. Das Gurtelement (210) umschlingt dann den Unterschenkel (2001) und ist mit dem zweiten Ende (212) im Bereich des Unterschenkels nahe dem Sprunggelenk an sich selbst festgeklettet.

Der hier gezeigte Verlauf des Gurtelements (210) entspricht also dem Verlauf des in den Figuren 1 bis 11 gezeigten Gurtelements an der Hand, nur dass das Gurtelement nicht im Zwischenraum zwischen dem großen Zeh und dem daneben liegenden Zeh verläuft, wobei auch eine solche Ausgestaltung möglich ist. Das Gurtelement (210) weist wieder elastische und nicht elastische Abschnitte auf, die in ihrer Positionierung in Bezug auf den Fuß und das Bein der Positionierung der oben gezeigten Abschnitte in Bezug auf die Hand und den Arm entsprechen. Beispielsweise ist der Abschnitt an der Fußsohle elastisch, sowie auch der zweite Endbereich des Gurtelements, die Abschnitte an den Fußseiten sind aber unelastisch. In der vorliegenden Ausführungsform ist das Gurtelement (210) einstückig und hat keine trennbaren Teilelemente. Der Fachmann kann alle übrigen Aspekte der beispielhaft gezeigten Handgelenksbandage (100) auf die Sprunggelenksbandage (200) übertragen.

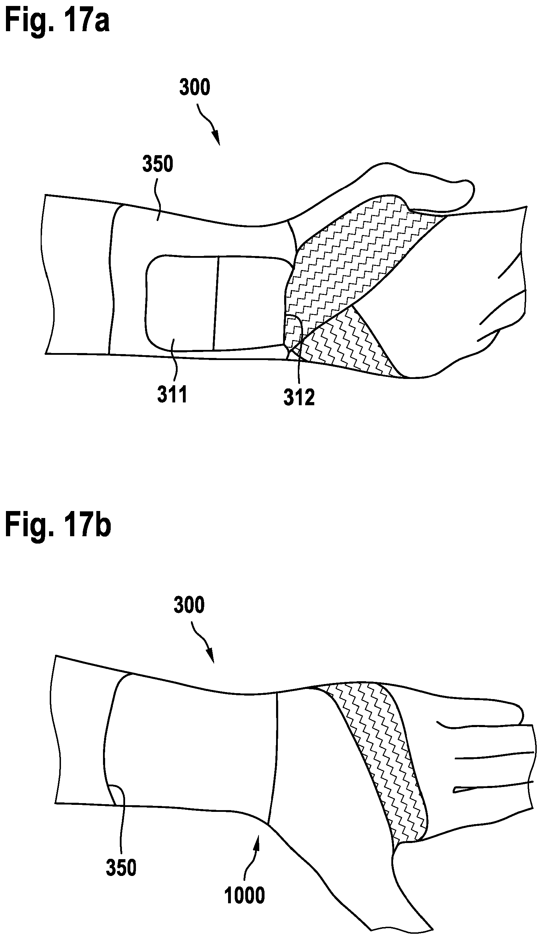

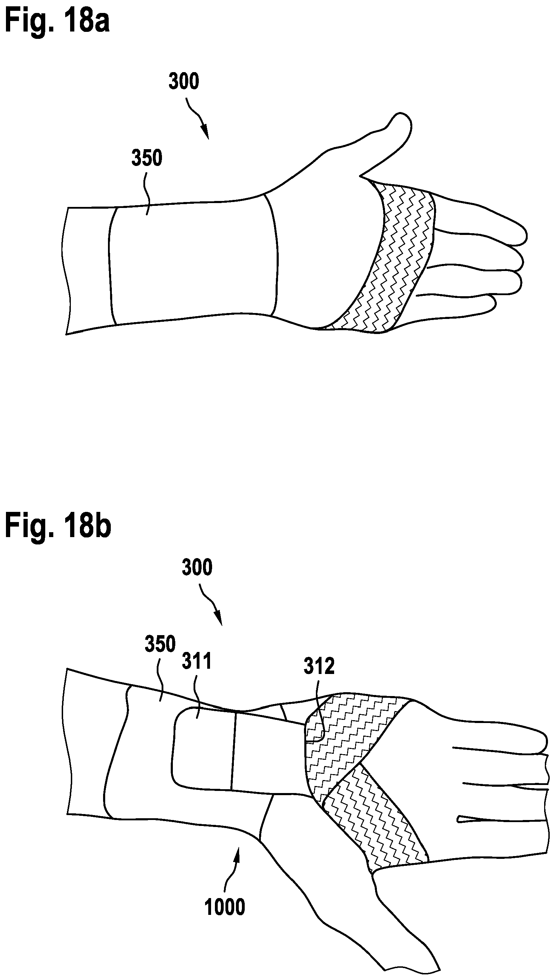

Die Figuren 17 und 18 zeigen schematisch eine weitere Ausführungsform der erfindungsgemäßen Handgelenksbandage (300) in der Ausführungsform mit kurzem Schlauchelement (350) an einem rechten Handgelenk (1000). An dem Schlauchelement (350) ist ein erfindungsgemäßes Gurtelement (310) befestigt. Figur 17a und 18a zeigen die Handgelenksbandage (300) am Handrücken und die Figuren 17b und 18b zeigen die Handgelenksbandage (300) an der Handinnenfläche. Das Gurtelement (310) weist ein erstes Ende (311) und ein zweites Ende (312) auf, wobei das erste Ende (311) des Gurtelements (310) am Schlauchelement (350) über eine Klettverbindung befestigt ist, wobei das zweite Ende (312) des Gurtelements (310) am Gurtelement (310) befestigt ist. Es ist auch bei dieser Bandage kein Stabilisierungsstab nötig.

Das Gurtelement (310) weist wieder elastische und nicht elastische Abschnitte auf, die in ihrer Positionierung in Bezug auf die Hand und den Arm grundsätzlich denen der Handgelenksbandage aus den Figuren 1 bis 11 entsprechen, auch wenn der Verlauf des Gurtelements unterschiedlich ist. Beispielsweise ist der Abschnitt an der Handinnenfläche in Figur 17b und am Handrücken in Figur 18a elastisch, sowie auch der zweite Endbereich des Gurtelements, die Abschnitte an den Handseiten sind aber unelastisch. In der vorliegenden Ausführungsform ist das Gurtelement (210) einstückig und hat keine trennbaren Teilelemente. Der Fachmann kann alle übrigen Aspekte der beispielhaft gezeigten Handgelenksbandage (100) auf die hier gezeigte Ausführungsform (300) übertragen.

## Patentansprüche

1. Gelenkbandage (100, 200) für ein Handgelenk (1000) oder ein Sprunggelenk (2000), umfassend ein Gurtelement (10, 210) mit einem ersten Ende (11, 211) und einem zweiten Ende (12, 212), wobei das Gurtelement (10, 210) mindestens zwei elastische Abschnitte (14, 16) und mindestens einen nicht elastischen Abschnitt (15) zwischen den zwei elastischen Abschnitten (14, 16) aufweist, wobei das zweite Ende (12, 212) des Gurtelements (10, 210) am Gurtelement (10, 210) oder an einem Schlauchelement (50, 250) befestigt oder reversibel befestigbar ist, wobei das Gurtelement (10, 210) aufeinanderfolgend einen ersten elastischen Abschnitt (14), einen ersten nicht elastischen Abschnitt (15), einen zweiten elastischen Abschnitt (16) und einen zweiten nicht elastischen Abschnitt (17) aufweist, wobei der erste nicht elastische Abschnitt (15) einen Abschnitt in Form eines Parallelogramms oder einer Krümmung oder einen Knick aufweist, **dadurch gekennzeichnet, dass** das Gurtelement (10, 210) aufeinanderfolgend, nach dem zweiten nicht elastischen Abschnitt (17), einen dritten elastischen Abschnitt (18) aufweist, und dass der zweite nicht elastische Abschnitt (17) eine Verjüngung aufweist.

2. Gelenkbandage (100, 200) nach Anspruch 1, wobei das Gurtelement (10, 210) einen Fixierungsabschnitt aufweist, der um das Zygopodium (1001, 2001), insbesondere den Unterarm (1001) oder den Unterschenkel (2001), verlaufen kann und der Fixierungsabschnitt zwei miteinander verbindbare Bereiche aufweist, so dass der Fixierungsabschnitt durch das Verbinden der zwei Bereiche am Zygopodium (1001, 2001), insbesondere am Unterarm (1001) oder am Unterschenkel (2001), fixierbar ist.

3. Gelenkbandage (100, 200) nach Anspruch 2, wobei das Gurtelement (10, 210) ein erstes Ende (11, 211) und ein zweites Ende (12, 212) aufweist, wobei das erste Ende (11, 211) des Gurtelements (10, 210) durch den Fixierungsabschnitt gebildet wird und am Zygopodium (1001, 2001) befestigbar ist, wobei das zweite Ende (12, 212) des Gurtelements (10, 210) am Gurtelement (10, 210) reversibel befestigbar ist, wobei im angelegten Zustand das erste Ende (11, 211) des Gurtelements (10, 210) durch den Fixierungsabschnitt am Zygopodium (1001, 2001) positioniert ist und das Gurtelement (10, 210) vom ersten Ende (11, 211) aus distal über das Extremitäten-Endgelenk (1000, 2000) zum Basipodium (1002, 2002) verläuft, Basipodium (1002, 2002) oder Metapodium (2003) auf der Innenseite umläuft, dorsal über das Handgelenk (1000) oder Sprunggelenk (2000) zum Zygopodium (1001, 2001) verläuft und sich dabei kreuzt, das Zygopodium (1001, 2001) umläuft und mit dem zweiten Ende (12, 212) im Bereich des Zygopodiums (1001, 2001) am Gurtelement (10, 210) reversibel befestigt ist.

4. Gelenkbandage nach einem der Ansprüche 1 oder 2, wobei das zweite Ende (12, 212) des Gurtelements (10, 210) am Gurtelement (10, 210) befestigt oder reversibel befestigbar ist.

5. Gelenkbandage (100, 200) für ein Handgelenk (1000) oder ein Sprunggelenk (2000) nach einem der vorhergehenden Ansprüche umfassend ein elastisches Schlauchelement (50, 250) und ein Gurtelement (10, 210), wobei das Schlauchelement (50, 250) eine erste Schlauchhälfte mit einem ersten Ende (51, 251) und eine zweite Schlauchhälfte mit einem zweiten Ende (52, 252) aufweist, wobei das Gurtelement (10, 210) ein erstes Ende (11, 211) und ein zweites Ende (12, 212) aufweist, wobei das erste Ende (11, 211) des Gurtelements (10, 210) an der ersten Schlauchhälfte (51, 251) befestigt ist oder befestigbar ist, wobei das zweite Ende (12, 212) des Gurtelements (10, 210) am Gurtelement (10, 210) oder an der ersten Schlauchhälfte (51, 251) reversibel befestigbar ist, wobei im angelegten Zustand das erste Ende des Gurtelements (10, 210) an der ersten Schlauchhälfte (51, 251) befestigt ist und am Zygopodium (1001, 2001) positioniert ist und das Gurtelement (10, 210) vom ersten Ende aus distal über das Extremitäten-Endgelenk (1000, 2000) zum Basipodium (1002, 2002) verläuft, Basipodium (1002, 2002) oder Metapodium (2003) auf der Innenseite umläuft, dorsal über das Extremitäten-Endgelenk (1000, 2000) zum Zygopodium (1001, 2001) verläuft und sich dabei kreuzt, das Zygopodium (1001, 2001) umläuft und mit dem zweiten Ende (12, 212) im Bereich des Zygopodiums (1001, 2001) am Gurtelement (10, 210) oder an der ersten Schlauchhälfte (51, 251) reversibel befestigt ist.

6. Gelenkbandage (100, 200) nach einem der vorhergehenden Ansprüche, wobei im angelegten Zustand mindestens ein elastischer Abschnitt des Gurtelements (10, 210) über dem Handrücken oder der Handfläche beziehungsweise über dem Fußrücken oder der Fußsohle verläuft und/oder wobei mindestens ein nicht elastischer Abschnitt an den Handkanten oder Fußkante verläuft.

7. Gelenkbandage (100, 200) nach einem der vorhergehenden Ansprüche, wobei das Gurtelement (10, 210) mindestens einen Abschnitt in Form eines Parallelogramms oder einer Krümmung oder einen Knick aufweist.

8. Gelenkbandage (100, 200) nach einem der vorhergehenden Ansprüche, wobei das Gurtelement (10, 210) sich abwechselnde elastische und nicht elastische Abschnitte aufweist.

9. Gelenkbandage (100, 200) nach einem der vorhergehenden Ansprüche, wobei dem ersten elastischen Abschnitt (14) noch ein nicht elastischer Vorabschnitt (13) zugeordnet ist.

10. Gelenkbandage (100, 200) nach einem der vorhergehenden Ansprüche, wobei das Gurtelement (10, 210) mindestens zwei reversibel miteinander verbindbare Gurtteilelemente umfasst.

11. Gelenkbandage (100, 200) nach einem der vorhergehenden Ansprüche, wobei das Gurtelement (10, 210) ein erstes Gurtteilelement mit dem ersten Ende (11, 211) und ein zweites Gurtteilelement mit dem zweiten Ende (12, 212) umfasst, wobei das zweite Gurtteilelement elastisch ist.

12. Gelenkbandage (100, 200) nach Anspruch 11, wobei das erste Gurtteilelement und das zweite Gurtteilelement reversibel über ein drittes Ende am ersten Gurtteilelement und ein viertes Ende am zweiten Gurtteilelement miteinander verbindbar sind und wobei bevorzugt das vierte Ende am zweiten Gurtteilelement ein Verbindungselement umfasst, mit dem das zweite Gurtteilelement ohne das erste Gurtteilelement am Schlauchelement (50, 250) reversibel befestigt werden kann.

13. Gelenkbandage (100, 200) nach einem der vorhergehenden Ansprüche, wobei die Gelenkbandage (100, 200) keinen Stabilisierungsstab aufweist.

## Claims

1. A joint bandage (100, 200) for a wrist (1000) or an ankle (2000), comprising a strap element (10, 210) with a first end (11, 211) and a second end (12, 212), wherein the strap element (10, 210) has at least two elastic sections (14, 16) and at least one non-elastic section (15) between the two elastic sections (14, 16), wherein the second end (12, 212) of the strap element (10, 210) is fastened or reversibly fastenable to the strap element (10, 210) or to a tube element (50, 250), wherein the strap element (10, 210) successively has a first elastic section (14), a first non-elastic section (15), a second elastic section (16), a second non-elastic section (17) and a third elastic section (18), wherein the first non-elastic section (15) has a section in the form of a parallelogram or a curvature or a kink, **characterised in that** the second non-elastic section (17) has a taper.

2. The joint bandage (100, 200) according to claim 1, wherein the strap element (10, 210) has a fixation section which can run around the zygopodium (1001, 2001), in particular the forearm (1001)or the lower leg (2001), and the fixation section has two interconnectable areas, so that the fixation section is fixable to the zygopodium (1001, 2001), in particular the forearm (1001) or the lower leg (2001), by connecting the two areas.

3. The joint bandage (100, 200) according to claim 2, wherein the strap element (10, 210) has a first end (11, 211) and a second end (12, 212), wherein the first end (11, 211) of the strap element (10, 210) is formed by the fixation section and is fastenable to the zygopodium (1001, 2001), wherein the second end (12, 212) of the strap element (10, 210) is reversibly fastenable to the strap element (10, 210), wherein, in the applied state, the first end (11, 211) of the strap element (10, 210) is positioned on the zygopodium (1001, 2001) by the fixation section and the strap element (10, 210) runs from the first end (11, 211) distally over the extremity end joint (1000, 2000) to the basipodium (1002, 2002), runs around the basipodium (1002, 2002) or metapodium (2003) on the inside, runs dorsally over the wrist (1000) or ankle (2000) to the zygopodium (1001, 2001) and thereby crosses itself, runs around the zygopodium (1001, 2001) and is reversibly fastened to the strap element (10, 210) with the second end (12, 212) in the area of the zygopodium (1001, 2001).

4. The joint bandage according to claim 1 or 2, wherein the second end (12, 212) of the strap element (10, 210) is fastened or reversibly fastenable to the strap element (10, 210).

5. A joint bandage (100, 200) for a wrist (1000) or an ankle (2000) according to one of the preceding claims comprising an elastic tube element (50, 250) and a strap element (10, 210), wherein the tube element (50, 250) has a first tube half with a first end (51, 251) and a second tube half with a second end (52, 252), wherein the strap element (10, 210) has a first end (11, 211) and a second end (12, 212), wherein the first end (11, 211) of the strap element (10, 210) is fastened or fastenable to the first tube half (51, 251), wherein the second end (12, 212) of the strap element (10, 210) is reversibly fastenable to the strap element (10, 210) or to the first tube half (51, 251), wherein, in the applied state, the first end of the strap element (10, 210) is fastened to the first tube half (51, 251) and is positioned on the zygopodium (1001, 2001) and the strap element (10, 210) runs from the first end distally over the extremity end joint (1000, 2000) to the basipodium (1002, 2002), runs around the basipodium (1002, 2002) or metapodium (2003) on the inside, runs dorsally over the extremity end joint (1000, 2000) to the zygopodium (1001, 2001) and thereby crosses itself, runs around the zygopodium (1001, 2001) and is reversibly fastened to the strap element (10, 210) or to the first tube half (51, 251) with the second end (12, 212) in the area of the zygopodium (1001, 2001).

6. The joint bandage (100, 200) according to one of the preceding claims, wherein in the applied state at least one elastic section of the strap element (10, 210) runs over the back of the hand or the palm of the hand or over the back of the foot or the sole of the foot and/or wherein at least one non-elastic section runs along the edges of the hand or the edge of the foot.

7. The joint bandage (100, 200) according to any one of the preceding claims, wherein the strap element (10, 210) has at least one section in the form of a parallelogram or a curvature or a kink.

8. The joint bandage (100, 200) according to any one of the preceding claims, wherein the strap element (10, 210) has alternating elastic and non-elastic sections.

9. The joint bandage (100, 200) according to any one of the preceding claims, wherein a non-elastic pre-section (13) is further associated to the first elastic section (14).

10. The joint bandage (100, 200) according to any one of the preceding claims, wherein the strap element (10, 210) comprises at least two reversibly interconnectable strap part elements.

11. The joint bandage (100, 200) according to any one of the preceding claims, wherein the strap element (10, 210) comprises a first strap part element with the first end (11, 211) and a second strap part element with the second end (12, 212), wherein the second strap part element is elastic.

12. The joint bandage (100, 200) according to claim 11, wherein the first strap part element and the second strap part element are reversibly interconnectable via a third end on the first strap part element and a fourth end on the second strap part element, and wherein preferably the fourth end on the second strap part element comprises a connecting element with which the second strap part is reversibly fastenable to the tube element (50, 250) without the first strap part element.

13. The joint bandage (100, 200) according to any one of the preceding claims, wherein the joint bandage (100, 200) has no stabilising bar.

## Revendications

1. Bandage d'articulation (100, 200) pour un poignet (1000) ou une cheville (2000), comportant un élément formant sangle (10, 210) avec une première extrémité (11, 211) et une deuxième extrémité (12, 212), dans lequel l'élément formant sangle (10, 210) comprend au moins deux sections élastiques (14, 16) et au moins une section non élastique (15) entre les deux sections élastiques (14, 16), dans lequel la deuxième extrémité (12, 212) de l'élément formant sangle (10, 210) est attachée ou peut être attachée de manière réversible sur l'élément formant sangle (10, 210) ou sur un élément tubulaire (50, 250), dans lequel l'élément formant sangle (10, 210) comprend successivement une première section élastique (14), une première section non élastique (15), une deuxième section élastique (16) et une deuxième section non élastique (17), dans lequel la première section non élastique (15) comprend une section sous la forme d'un parallélogramme ou d'un coude ou d'un pli, **caractérisé en ce que** l'élément formant sangle (10, 210) comprend successivement, après la deuxième section non élastique (17), une troisième section élastique (18), et **en ce que** la deuxième section non élastique (17) présente un rétrécissement.

2. Bandage d'articulation (100, 200) selon la revendication 1, dans lequel l'élément formant sangle (10, 210) comprend une section de fixation qui peut s'étendre autour du zeugopode (1001, 2001), en particulier de l'avant-bras (1001) ou de la jambe (2001), et la section de fixation comprend deux zones pouvant être reliées l'une à l'autre de telle sorte que la section de fixation puisse être fixée par le biais de la liaison des deux zones sur le zeugopode (1001, 2001), en particulier sur l'avant-bras (1001) ou sur la jambe (2001).

3. Bandage d'articulation (100, 200) selon la revendication 2, dans lequel l'élément formant sangle (10, 210) comprend une première extrémité (11, 211) et une deuxième extrémité (12, 212), dans lequel la première extrémité (11, 211) de l'élément formant sangle (10, 210) est formée par le biais de la section de fixation et peut être attachée sur le zeugopode (1001, 2001), dans lequel la deuxième extrémité (12, 212) de l'élément formant sangle (10, 210) peut être attachée de manière réversible sur l'élément formant sangle (10, 210), dans lequel, à l'état mis en place, la première extrémité (11, 211) de l'élément formant sangle (10, 210) est positionnée par le biais de la section de fixation sur le zeugopode (1001, 2001) et l'élément formant sangle (10, 210) s'étend depuis la première extrémité (11, 211) de manière distale par-dessus l'articulation d'extrémité de membre (1000, 2000) jusqu'au basipode (1002, 2002), fait le tour du basipode (1002, 2002) ou du métapode (2003) sur la face interne, s'étend de manière dorsale par-dessus le poignet (1000) ou la cheville (2000) jusqu'au zeugopode (1001, 2001) et s'y croise, fait le tour du zeugopode (1001, 2001) et est attaché de manière réversible par la deuxième extrémité (12, 212) dans la zone du zeugopode (1001, 2001) sur l'élément formant sangle (10, 210).

4. Bandage d'articulation selon l'une des revendications 1 ou 2, dans lequel la deuxième extrémité (12, 212) de l'élément formant sangle (10, 210) est attachée ou peut être attachée de manière réversible sur l'élément formant sangle (10, 210).

5. Bandage d'articulation (100, 200) pour un poignet (1000) ou une cheville (2000) selon l'une des revendications précédentes comprenant un élément tubulaire (50, 250) élastique et un élément formant sangle (10, 210), dans lequel l'élément tubulaire (50, 250) comprend une première moitié de tube avec une première extrémité (51, 251) et une deuxième moitié de tube avec une deuxième extrémité (52, 252), dans lequel l'élément formant sangle (10, 210) comprend une première extrémité (11, 211) et une deuxième extrémité (12, 212), dans lequel la première extrémité (11, 211) de l'élément formant sangle (10, 210) est attachée ou peut être attachée sur la première moitié de tube (51, 251), dans lequel la deuxième extrémité (12, 212) de l'élément formant sangle (10, 210) peut être attachée de manière réversible sur l'élément formant sangle (10, 210) ou sur la première moitié de tube (51, 251), dans lequel, à l'état mis en place, la première extrémité de l'élément formant sangle (10, 210) est attachée sur la première moitié de tube (51, 251) et est positionnée sur le zeugopode (1001, 2001) et l'élément formant sangle (10, 210) s'étend depuis la première extrémité de manière distale par-dessus l'articulation d'extrémité de membre (1000, 2000) jusqu'au basipode (1002, 2002), fait le tour du basipode (1002, 2002) ou du métapode (2003) sur la face interne, s'étend de manière dorsale par-dessus l'articulation d'extrémité de membre (1000, 2000) jusqu'au zeugopode (1001, 2001) et s'y croise, fait le tour du zeugopode (1001, 2001) et est attaché de manière réversible par la deuxième extrémité (12, 212) dans la zone du zeugopode (1001, 2001) sur l'élément formant sangle (10, 210) ou sur la première moitié de tube (51, 251).

6. Bandage d'articulation (100, 200) selon l'une des revendications précédentes, dans lequel, à l'état mis en place, au moins une section élastique de l'élément formant sangle (10, 210) s'étend par-dessus le dos de la main ou la paume de la main, respectivement par-dessus le dessus du pied ou la plante du pied, et/ou dans lequel au moins une section non élastique s'étend sur les bords de la main ou le bord du pied.

7. Bandage d'articulation (100, 200) selon l'une des revendications précédentes, dans lequel l'élément formant sangle (10, 210) comprend au moins une section sous la forme d'un parallélogramme ou d'un coude ou d'un pli.

8. Bandage d'articulation (100, 200) selon l'une des revendications précédentes, dans lequel l'élément formant sangle (10, 210) comprend des sections élastiques et non élastiques alternées.

9. Bandage d'articulation (100, 200) selon l'une des revendications précédentes, dans lequel encore une section avant non élastique (13) est associée à la première section élastique (14).

10. Bandage d'articulation (100, 200) selon l'une des revendications précédentes, dans lequel l'élément formant sangle (10, 210) comprend au moins deux éléments partiels de sangle pouvant être reliés l'un à l'autre de manière réversible.

11. Bandage d'articulation (100, 200) selon l'une des revendications précédentes, dans lequel l'élément formant sangle (10, 210) comprend un premier élément partiel de sangle avec la première extrémité (11, 211) et un deuxième élément partiel de sangle avec la deuxième extrémité (12, 212), dans lequel le deuxième élément partiel de sangle est élastique.

12. Bandage d'articulation (100, 200) selon la revendication 11, dans lequel le premier élément partiel de sangle et le deuxième élément partiel de sangle peuvent être reliés l'un à l'autre de manière réversible par l'intermédiaire d'une troisième extrémité sur le premier élément partiel de sangle et d'une quatrième extrémité sur le deuxième élément partiel de sangle et dans lequel, de préférence, la quatrième extrémité comprend, sur le deuxième élément partiel de sangle, un élément de liaison par lequel le deuxième élément partiel de sangle peut être attaché de manière réversible sans le premier élément partiel de sangle sur l'élément tubulaire (50, 250).

13. Bandage d'articulation (100, 200) selon l'une des revendications précédentes, le bandage d'articulation (100, 200) ne comprenant pas de tige de stabilisation.
